# EUROPÄISCHE PATENTSCHRIFT

(11) **EP 2 765 929 B1**
(45) Veröffentlichungstag und Bekanntmachung des Hinweises auf die Patenterteilung: **13.01.2021**
(21) Anmeldenummer: 12783874.6
(22) Anmeldetag: 12.10.2012
(51) Int. Cl.: A61B 17/3205, A61B 17/16

(54) **CHIRURGIEWERKZEUG**
SURGICAL TOOL
INSTRUMENT CHIRURGICAL

(30) Priorität: 14.10.2011 DE 202011106747 U
(43) Veröffentlichungstag der Anmeldung: 20.08.2014
(73) Patentinhaber: DANNORITZER Medizintechnik GmbH & Co. KG, 78532 Tuttlingen (DE)
(72) Erfinder: DANNORITZER, Axel, 78532 Tuttlingen (DE)
(74) Vertreter: Daub, Thomas
(86) Internationale Anmeldenummer: PCT/EP2012/004279
(87) Internationale Veröffentlichungsnummer: WO 2013/053490

(56) Entgegenhaltungen:
- WO-A1-00/45714
- WO-A2-2005/051245
- DD-A1- 103 386
- DE-A1- 2 602 238
- DE-A1- 3 831 046
- DE-A1- 4 208 089
- GB-A- 2 138 332
- US-A- 5 312 408
- US-A1- 2006 173 476
- US-A1- 2007 212 179
- US-B1- 7 189 036

## Beschreibung

### Stand der Technik

Die Erfindung geht aus von einem Chirurgiewerkzeug nach dem Oberbegriff des Anspruchs 1.

Es sind bereits Chirurgiewerkzeuge mit einem Grundkörper, mit einem Kopplungselement und mit einer Bearbeitungseinheit bekannt, wobei die Bearbeitungseinheit eine Vielzahl von Schneidelementen, die insbesondere von Diamantsplittern gebildet sind, aufweist. Die von Diamantsplittern gebildeten Schneidelemente sind dabei in einer Trägerschicht eingebettet und darin gehalten. Die Schneidelemente erstrecken sich dabei über eine gesamte Fläche der Bearbeitungseinheit.

DE 42 08 089 A1, US 5,312,408 A, DD 103 386 A1, WO2005/051245 A2, US 2006/0173476 A1 offenbaren Chirurgiewerkzeuge mit einem hohlzylindrischen Grundkörper und einer distalen Bearbeitungseinheit. DE 42 08 089 A1 offenbart eine Bearbeitungseinheit mit einem distalen Bearbeitungsbereich und einem Freischneidbereich der sich in Radialrichtung vom Grundkörper nach Außen erstreckt.

### Vorteile der Erfindung

Ein erfindungsgemäßes Chirurgiewerkzeug wird durch die Merkmale des unabhängigen Anspruchs definiert. Weitere vorteilhafte Ausgestaltungen sind in den abhängigen Ansprüchen angegeben.

Die Erfindung geht aus von einem Chirurgiewerkzeug mit einem Grundkörper, mit einem Kopplungselement und mit einer Bearbeitungseinheit, wobei die Bearbeitungseinheit zumindest einen Bearbeitungsbereich und zumindest einen Freischneidbereich umfasst.

Es wird vorgeschlagen, dass die Bearbeitungseinheit zumindest zwei segmentweise angeordnete Schneidelemente aufweist. Unter einem "Chirurgiewerkzeug" soll in diesem Zusammenhang insbesondere ein Werkzeug, vorzugsweise ein Einsatzwerkzeug, verstanden werden, dass dazu vorgesehen ist, insbesondere bei zumindest einem chirurgischen Eingriff, vorzugsweise bei zumindest einer Operation an einem Lebewesen, vorzugsweise an einem Menschen, verwendet zu werden. Das Chirurgiewerkzeug kann von einem Bohr-, Säge- und/oder Trennwerkzeug gebildet sein. Es sind auch weitere, einem Fachmann als sinnvoll erscheinende Ausgestaltungen des Chirurgiewerkzeugs denkbar. Unter "vorgesehen" soll insbesondere speziell ausgelegt, ausgestattet und/oder ausgestattet verstanden werden. Unter einem "Kopplungselement" soll in diesem Zusammenhang insbesondere ein Element verstanden werden, dass dazu vorgesehen ist, das Chirurgiewerkzeug insbesondere mit einer Chirurgiehandwerkzeugmaschine vorzugsweise lösbar und/oder zumindest im Wesentlichen werkzeuglos zu verbinden. Unter "lösbar" soll in diesem Zusammenhang insbesondere verstanden werden, dass das Chirurgiewerkzeug von einem Bediener vorzugsweise werkzeuglos entnommen werden kann. Das Kopplungselement kann als wiederverwendbares Kopplungselement ausgestaltet sein. Alternativ kann das Kopplungselement so ausgestaltet sein, dass es bei einem Lösen von der Chirurgiehandwerkzeugmaschine derart verformt und/oder verändert wird, dass ein erneutes Verbinden des Kopplungselements mit der Chirurgiehandwerkzeugmaschine verhindert werden kann. Das Koppelelement kann insbesondere von dem in der DE 10 2010 049 244.2 beschriebenen Koppelelement gebildet sein. Es sind jedoch auch alternative, einem Fachmann als sinnvoll erscheinende Ausgestaltungen des Koppelmittels denkbar. Unter einer "Bearbeitungseinheit" soll in diesem Zusammenhang insbesondere eine Einheit verstanden werden, die dazu vorgesehen ist, in einem Betriebszustand des Chirurgiewerkzeugs ein zu bearbeitendes Material, wie insbesondere ein Knochen, Haut, einen Muskel und/oder ein anderes, einem Fachmann als sinnvoll erscheinendes Gewebe vorzugsweise direkt zu kontaktieren und insbesondere formändernd zu bearbeiten. Unter einem "Freischneidbereich" soll in diesem Zusammenhang insbesondere ein Bereich verstanden werden, der sich zumindest im Wesentlichen in Radialrichtung über den Grundkörper des Chirurgiewerkzeugs hinaus erstreckt und der dazu vorgesehen ist, einen senkrecht zur Bearbeitungsachse verlaufenden Abstand zwischen zumindest einem Großteil des Grundkörpers und dem zu bearbeitenden Material zumindest im Wesentlichen sicherzustellen. In einem besonders bevorzugten Ausführungsbeispiel ist der Freischneidbereich dazu vorgesehen, in einem Bearbeitungszustand zwischen einer Oberfläche des Grundkörpers und dem Material, das mit dem Chirurgiewerkzeug bearbeitet wird, einen Abstand insbesondere durch ein Abtragen einer Materialschicht zu erreichen. Unter "segmentweise angeordnet" soll in diesem Zusammenhang insbesondere sortiert und gezielt voneinander beabstandet und vorzugsweise zumindest im Wesentlichen gleichmäßig verteilt verstanden werden. Vorzugsweise weisen die segmentweise angeordneten Schneidelemente einen Abstand zueinander von zumindest 1°, insbesondere von zumindest 10°, vorzugsweise von zumindest 20° und besonders bevorzugt von zumindest 45° auf. Unter einem "Schneidelement" soll in diesem Zusammenhang insbesondere ein Element verstanden werden, das dazu vorgesehen ist, ein Material, insbesondere einen Knochen und/oder ein anderes einem Fachmann als sinnvoll erscheinendes, vorzugsweise menschliches Gewebe, zu trennen.

Dadurch kann ein vorteilhaft präzises Bearbeitungsergebnis erreicht werden. Insbesondere durch die erfindungsgemäße Ausgestaltung des Freischneidbereichs kann eine unerwünschte Erhitzung des zu bearbeitenden Materials während eines Bearbeitungszustands auf vorteilhaft einfache Weise sicher verhindert werden. Insbesondere durch die gezielte, segmentweise Anordnung der zumindest zwei Schneidelemente können auf konstruktiv einfache Weise vorteilhaft Material, aus dem die Schneidelemente gebildet sind, und somit bevorzugt Kosten eingespart werden, wobei eine negative Auswirkung auf das Bearbeitungsergebnis sicher verhindert werden kann.

Ferner wird vorgeschlagen, dass die Bearbeitungseinheit maximal 50 Schneidelemente umfasst. Dadurch können auf konstruktiv einfache Weise vorteilhaft Material, aus dem die Schneidelemente gebildet sind, und somit bevorzugt Kosten eingespart werden.

Zudem wird vorgeschlagen, dass die zumindest zwei Schneidelemente zumindest im Wesentlichen aus zumindest einem Hartmetall gebildet sind. Unter einem "Hartmetall" soll in diesem Zusammenhang insbesondere ein Metall verstanden werden, das sich aus zumindest einem harten und spröden Metall und aus zumindest einem weicheren Metall, insbesondere einem sogenannten Bindemetall, zusammensetzt. In einem besonders bevorzugten Ausführungsbeispiel weisen die aus einem Hartmetall gebildeten Schneidelemente eine größere Härte auf als ein Material des Grundkörpers und/oder eines weiteren Materials, mit dem die Schneidelemente insbesondere formschlüssig verbunden sind. Vorzugsweise kann das Hartmetall einen Anteil von zumindest 50 % an zumindest einem Carbid, insbesondere an Wolframcarbid, und einen Anteil von zumindest 5 % insbesondere an Cobalt aufweisen. Es sind auch andere einem Fachmann als sinnvoll erscheinende Zusammensetzungen eines Hartmetalls denkbar. Durch die erfindungsgemäße Ausgestaltung kann eine vorteilhaft einfache Reinigung des Chirurgiewerkzeugs erreicht werden. Durch die Verwendung eines Hartmetalls können zudem vorteilhaft Kosten eingespart werden.

In einer weiteren Ausgestaltung wird vorgeschlagen, dass die zumindest zwei Schneidelemente zumindest im Wesentlichen aus zumindest einem Keramikwerkstoff gebildet sind. Unter einem "Keramikwerkstoff" soll in diesem Zusammenhang insbesondere ein anorganischer, nichtmetallischer, polykristalliner Stoff verstanden werden, der insbesondere der DIN EN 12 212 entspricht. Die Schneidelemente können auch von Diamantstücken gebildet sein, die mit dem Grundkörper des Chirurgiewerkzeugs insbesondere verklebt sein können. So kann eine flexible Anpassung des Materials der Schneidelemente an einen jeweiligen Anwendungsfall des Chirurgiewerkzeugs erreicht werden.

Zudem wird vorgeschlagen, dass die zumindest zwei Schneidelemente von Schneidzähnen gebildet sind, die zumindest teilweise geschränkt angeordnet sind. Durch die zumindest teilweise Schränkung der als Schneidzähne ausgebildeten Schneidelemente kann auf konstruktiv einfache Weise der Freischneidbereich gebildet sein. Durch die Ausbildung der Schneidelemente als Schneidzähne könnte beispielsweise eine zumindest im Wesentlichen einstückige Ausgestaltung des Chirurgiewerkzeugs erreicht werden. Dadurch könnte eine vorteilhaft einfache und kostensparende Ausgestaltung des Chirurgiewerkzeugs erreicht werden.

Des Weiteren wird vorgeschlagen, dass die Schneidelemente zumindest im Wesentlichen stoffschlüssig mit dem Grundkörper verbunden sind. Unter "stoffschlüssig verbunden" soll insbesondere verstanden werden, dass die Masseteile durch atomare oder molekulare Kräfte zusammengehalten werden, wie beispielsweise durch Löten, Schweißen, Kleben und/oder Vulkanisieren.

Ferner wird vorgeschlagen, dass die Schneidelemente zumindest im Wesentlichen mit dem Grundkörper verschweißt sind. Dadurch kann eine bevorzugt robuste Ausgestaltung des Chirurgiewerkzeugs erreicht werden.

Des Weiteren wird vorgeschlagen, dass die Schneidelemente zumindest teilweise von dem Grundkörper umgeben sind. Unter "zumindest teilweise umgeben" soll in diesem Zusammenhang insbesondere verstanden werden, dass das Schneidelement von dem Grundkörper zu zumindest 10 %, vorzugsweise zu zumindest 25 % und besonders bevorzugt zu zumindest 50 % umschlossen ist. Dadurch kann eine vorteilhaft robuste und langlebige Ausgestaltung des Chirurgiewerkzeugs erreicht werden.

Zudem wird vorgeschlagen, dass der Grundkörper zumindest im Wesentlichen von einem Rohr gebildet ist. Unter einem "Rohr" soll in diesem Zusammenhang insbesondere ein hohlzylindrisch ausgebildeter Körper verstanden werden. Dadurch kann eine vorteilhaft schmale und insbesondere für rotatorisch angetriebene Chirurgiewerkzeuge geeignete Ausbildung des Chirurgiewerkzeugs erreicht werden.
Ferner wird vorgeschlagen, dass die Schneidelemente zumindest im Wesentlichen gleichmäßig über einen Umfang des Grundkörpers verteilt mit dem Grundkörper verbunden sind. Dadurch können eine vorteilhaft gleichmäßige Bearbeitung und somit ein bevorzugt präzises Bearbeitungsergebnis erreicht werden.

Ferner wird ein Verfahren zur Herstellung des erfindungsgemäßen Chirurgiewerkzeugs vorgeschlagen, wobei zumindest zwei Schneidelemente einer Bearbeitungseinheit segmentweise über einen Umfang eines Grundkörpers verteilt angeordnet und mit dem Grundkörper zumindest im Wesentlichen stoffschlüssig verbunden werden. Vorzugsweise wird dabei in zumindest einem ersten Schritt zumindest ein Schneidelement zumindest im Wesentlichen stoffschlüssig mit einem Grundkörper verbunden und in zumindest einem weiteren Schritt zumindest ein weiteres Schneidelement in zumindest einer Umfangsrichtung beabstandet zu dem zumindest einen ersten Schneidelement zumindest im Wesentlichen stoffschlüssig mit einem Grundkörper verbunden. In einem besonders bevorzugten Ausführungsbeispiel sind die Schneidelemente zumindest teilweise aus einem Hartmetall gebildet und werden insbesondere einzeln mit dem Grundkörper des Chirurgiewerkzeugs verschweißt. Vorzugsweise werden die Schneidelemente derart an dem Grundkörper angeordnet, dass in Axialrichtung ein Bearbeitungsbereich und in Radialrichtung ein Freischneidbereich entsteht.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die Erfindung wird durch die Ansprüche definiert.

Es zeigen:
- Fig. 1: ein erfindungsgemäßes Werkzeug in einer perspektivischen Ansicht,
- Fig. 2: Ausschnitt II des erfindungsgemäßen Werkzeugs in einer perspektivischen Ansicht,

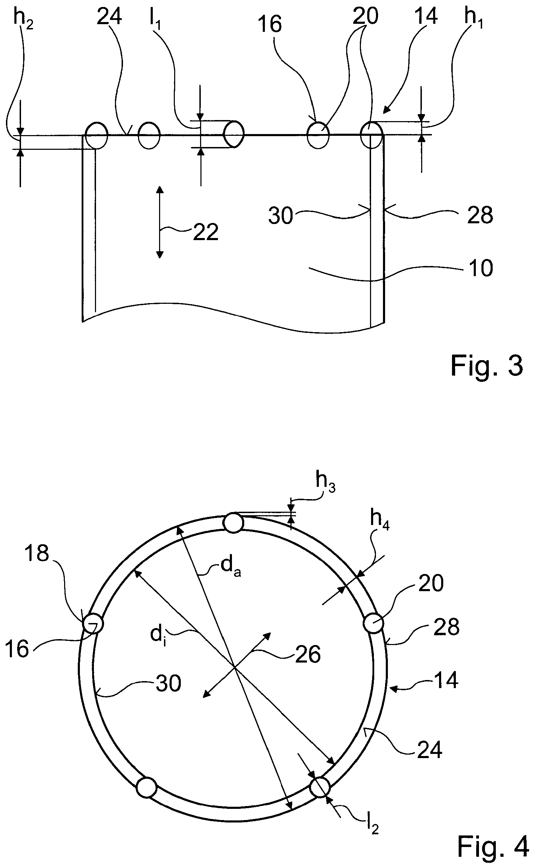

- Fig. 3: Ausschnitt II des erfindungsgemäßen Werkzeugs in einer Seitenansicht und
- Fig. 4.: das erfindungsgemäße Werkzeug in einer Frontalansicht.

### Beschreibung des Ausführungsbeispiels

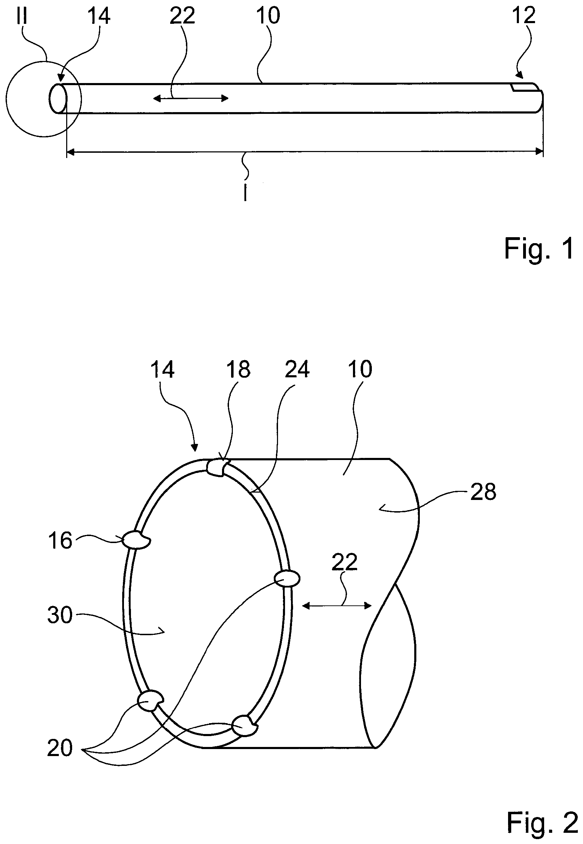

Figur 1 zeigt ein Chirurgiewerkzeug, das als chirurgischer Bohrer ausgebildet ist. Das Chirurgiewerkzeug umfasst einen Grundkörper 10. Der Grundkörper 10 weist eine längliche, zylindrische Form auf. Der Grundkörper 10 ist von einem metallischen Rohr gebildet. Der Grundkörper 10 ist aus einem Edelstahl gebildet. In einer Axialrichtung 22 des Chirurgiewerkzeugs betrachtet, ist an einem Ende des Grundkörpers 10 ein Kopplungselement 12 angeordnet. Das Kopplungselement 12 ist dazu vorgesehen, das Chirurgiewerkzeug mit einer nicht dargestellten Chirurgiewerkzeugmaschine verliersicher verbinden zu können. Das Kopplungselement 12 ist dazu vorgesehen, das Chirurgiewerkzeug mit einer Antriebswelle der Chirurgiewerkzeugmaschine insbesondere drehfest und lösbar zu verbinden.

Das Chirurgiewerkzeug ist dazu vorgesehen, bei einer Operation in einen insbesondere menschlichen Knochen hineinzubohren. Dabei entsteht eine kreisringförmige Bearbeitungsstelle, wobei senkrecht zu dieser kreisringförmigen Bearbeitungsstelle Material durch das Chirurgiewerkzeug bzw. die Schneidelemente 20 abgetragen wird. Dadurch entsteht ein zylinderförmiger Stift, der am Ende eines Bearbeitungsvorgangs aus dem restlichen Material herausgelöst werden kann. Im bearbeiteten Material bleibt ein zylinderförmiger Hohlraum zurück. Der Stift weist einen kleineren Durchmesser auf als der Hohlraum. Der Stift kann nun dazu genutzt werden, beispielsweise eine verletzte Sehne sicher am Knochen zu fixieren, indem der Stift zusammen mit einem Ende der Sehne in den Hohlraum in dem von Knochen gebildeten Material gesteckt wird. Dadurch kann eine vorteilhafte Fixierung der Sehne ohne einen Einsatz körperfremder Gegenstände, die zu Komplikationen führen können, erreicht werden.

An einem dem Kopplungselement 12 in Axialrichtung 22 gegenüberliegenden Ende des Grundkörpers 10 ist eine Bearbeitungseinheit 14 angeordnet. Die Bearbeitungseinheit 14 umfasst einen Bearbeitungsbereich 16 und einen Freischneidbereich 18 (Figur 2). Der Bearbeitungsbereich 16 erstreckt sich in Axialrichtung 22 an einer Stirnseite 24 über einen gesamten Umfang des Grundkörpers 10. Die Stirnseite 24 des Grundkörpers 10 ist ringförmig ausgebildet und weist eine Flächennormale auf, die sich parallel zur Axialrichtung 22 des Chirurgiewerkzeugs erstreckt. Der Bearbeitungsbereich 16 weist denselben Außendurchmesser auf wie der Grundkörper 10 des Chirurgiewerkzeugs. Der Bearbeitungsbereich 16 weist denselben Innendurchmesser auf wie der Grundkörper 10 des Chirurgiewerkzeugs. Der Freischneidbereich 18 der Bearbeitungseinheit 14 erstreckt sich in einer Radialrichtung 26 an einer Mantelaußenseite 28 und an einer Mantelinnenseite 30 über einen gesamten Umfang des Grundkörpers 10. Eine Flächennormale des Freischneidbereichs 18 erstreckt sich senkrecht zur Axialrichtung 22 des Chirurgiewerkzeugs. Der Freischneidbereich 18 weist einen größeren Außendurchmesser auf als der Grundkörper 10. Der Freischneidbereich 18 weist einen kleineren Innendurchmesser auf als der Grundkörper 10.

Die Bearbeitungseinheit 14 umfasst fünf Schneidelemente 20. Die Schneidelemente 20 sind segmentweise angeordnet. Die Schneidelemente 20 sind gleichmäßig über den Umfang des Grundkörpers 10 verteilt angeordnet. Die Schneidelemente 20 sind aus einem Hartmetall gebildet. Die Schneidelemente 20 sind stoffschlüssig mit dem Grundkörper 10 verbunden. Die Schneidelemente 20 sind mit dem Grundkörper 10 verschweißt. Die Schneidelemente 20 sind zumindest im Wesentlichen ellipsoidenförmig ausgebildet. Die Schneidelemente 20 weisen eine erste Länge l₁, die sich in einem mit dem Grundkörper 10 verbundenen Zustand parallel zur Axialrichtung 22 erstreckt, von zumindest im Wesentlichen 0,7 mm auf. In einem mit dem Grundkörper 10 verschweißten Zustand weisen die Schneidelemente 20 eine Höhe h₁ auf. In einem mit dem Grundkörper 10 verschweißten Zustand ragen die Schneidelemente 20 in Axialrichtung 22 entlang der Höhe h₁, die zumindest im Wesentlichen 0,45 mm beträgt, über die Stirnseite 24 des Grundkörpers 10 hinaus. Die Schneidelemente 20 weisen eine zweite Länge l₂, die sich in einem mit dem Grundkörper 10 verbundenen Zustand senkrecht zur Axialrichtung 22 erstreckt, von zumindest im Wesentlichen 0,6 mm auf. Der Bearbeitungsbereich 16 und der Freischneidbereich 18 sind zumindest im Wesentlichen von den Schneidelementen 20 gebildet. Die Schneidelemente 20 sind zumindest teilweise von dem Grundkörper 10 umgeben. Die Schneidelemente 20 sind entlang der ersten Länge l₁ betrachtet teilweise in dem Grundkörper 10 eingebettet. Die Schneidelemente 20 weisen in einem montierten Zustand eine parallel zur Axialrichtung 22 verlaufende Höhe h₂ auf, wobei das Schneidelement 20 entlang dieser Höhe h₂ von dem Grundkörper 10 umschlossen ist. Die Höhe h₂ beträgt zumindest im Wesentlichen 0,25 mm.

Der Grundkörper weist einen Außendurchmesser dₐ von zumindest im Wesentlichen 4,5 mm auf. Der Grundkörper 10 weist einen Innendurchmesser d, von zumindest im Wesentlichen 4,12 mm auf. Der Grundkörper 10 weist eine Wandstärke h₄ von zumindest im Wesentlichen 0,38 mm auf. Der Grundkörper 10 weist eine Gesamtlänge l in Axialrichtung 22 von zumindest im Wesentlichen 180 mm auf. Der Innendurchmesser des Freischneidbereichs 18 ist um eine Länge h₃ kleiner ausgestaltet als der Innendurchmesser dᵢ des Grundkörpers 10. Die Länge h₃ beträgt zumindest im Wesentlichen 1,1 mm. Der Außendurchmesser des Freischneidbereichs 18 ist um die Länge h₃ größer ausgestaltet als der Außendurchmesser dₐ des Grundkörpers 10.

**Bezugszeichen**

| | | | |
|---|---|---|---|
| 10 | Grundkörper | 30 | Mantelinnenseite |
| 12 | Kopplungselement | l | Gesamtlänge |
| 14 | Bearbeitungseinheit | l₁ | Länge |
| 16 | Bearbeitungsbereich | l₂ | Länge |
| 18 | Freischneidbereich | dᵢ | Innendurchmesser |
| 20 | Schneidelement | dₐ | Außendurchmesser |
| 22 | Axialrichtung | h₁ | Höhe |
| 24 | Stirnseite | h₂ | Höhe |
| 26 | Radialrichtung | h₃ | Länge |
| 28 | Mantelaußenseite | h₄ | Wandstärke |

## Patentansprüche

1. Chirurgiewerkzeug mit einem zumindest im Wesentlichen von einem hohlzylindrisch ausgebildeten Körper gebildeten Grundkörper (10), mit einem Kopplungselement (12) und mit einer Bearbeitungseinheit (14), wobei die Bearbeitungseinheit (14) zumindest einen sich in eine Axialrichtung (22) erstreckenden Bearbeitungsbereich (16) und zumindest einen Freischneidbereich (18) umfasst, der sich zumindest im Wesentlichen in eine Radialrichtung (26) über den Grundkörper (10) hinaus erstreckt und der dazu vorgesehen ist, einen senkrecht zu einer Bearbeitungsachse verlaufenden Abstand zwischen zumindest einem Großteil des Grundkörpers (10) und dem zu bearbeitenden Material zumindest im Wesentlichen sicherzustellen, **dadurch gekennzeichnet, dass** die Bearbeitungseinheit (14) zumindest zwei segmentweise angeordnete Schneidelemente (20) aufweist, die eine Länge l₁ aufweisen, die sich in einem mit dem Grundkörper (10) verbundenen Zustand parallel zur Axialrichtung (22) erstreckt und entlang deren die Schneidelemente (20) teilweise in dem Grundkörper (10) eingebettet sind

2. Chirurgiewerkzeug nach Anspruch 1,
**dadurch gekennzeichnet,**
**dass** die Bearbeitungseinheit (14) maximal 50 Schneidelemente (20) umfasst.

3. Chirurgiewerkzeug nach Anspruch 1 oder 2,
**dadurch gekennzeichnet,**
**dass** die zumindest zwei Schneidelemente einen Abstand zueinander von zumindest 10 Grad aufweisen.

4. Chirurgiewerkzeug zumindest nach Anspruch 1,
**dadurch gekennzeichnet,**
**dass** die zumindest zwei Schneidelemente (20) aus einem härteren Material als der Grundkörper (10) gebildet sind.

5. Chirurgiewerkzeug zumindest nach Anspruch 1,
**dadurch gekennzeichnet,**
**dass** die zumindest zwei Schneidelemente (20) zumindest im Wesentlichen aus zumindest einem Hartmetall gebildet sind.

6. Chirurgiewerkzeug zumindest nach Anspruch 1,
**dadurch gekennzeichnet,**
**dass** die zumindest zwei Schneidelemente (20) zumindest im Wesentlichen aus zumindest einem Keramikwerkstoff gebildet sind.

7. Chirurgiewerkzeug zumindest nach Anspruch 1,
**dadurch gekennzeichnet,**
**dass** die zumindest zwei Schneidelemente (20) zumindest im Wesentlichen ellipsoidenförmig ausgebildet sind.

8. Chirurgiewerkzeug zumindest nach Anspruch 1,
**dadurch gekennzeichnet,**
**dass** die zumindest zwei Schneidelemente (20) von Schneidzähnen gebildet sind, die zumindest teilweise geschränkt angeordnet sind.

9. Chirurgiewerkzeug nach zumindest einem der vorhergehenden Ansprüche,
**dadurch gekennzeichnet,**
**dass** die Schneidelemente (20) zumindest im Wesentlichen stoffschlüssig mit dem Grundkörper (10) verbunden sind.

10. Chirurgiewerkzeug nach zumindest einem der vorhergehenden Ansprüche,
**dadurch gekennzeichnet,**
**dass** die Schneidelemente (20) zumindest im Wesentlichen mit dem Grundkörper (10) verschweißt sind.

11. Chirurgiewerkzeug nach zumindest einem der vorhergehenden Ansprüche,
**dadurch gekennzeichnet,**
**dass** die Schneidelemente (20) zumindest teilweise von dem Grundkörper (10) umgeben sind.

12. Chirurgiewerkzeug nach zumindest einem der vorhergehenden Ansprüche,
**dadurch gekennzeichnet,**
**dass** der Grundkörper (10) zumindest im Wesentlichen von einem Rohr gebildet ist.

13. Chirurgiewerkzeug nach zumindest einem der vorhergehenden Ansprüche,
**dadurch gekennzeichnet,**
**dass** die Schneidelemente (20) zumindest im Wesentlichen gleichmäßig über einen Umfang des Grundkörpers (10) verteilt mit dem Grundkörper (10) verbunden sind.

## Claims

1. Surgical tool with a base body (10) that is at least substantially implemented of a hollow-cylindrically shaped body, with a coupling element (12) and with a processing unit (14), the processing unit (14) comprising at least one processing region (16) that extends in an axial direction (22) and at least one cut-out region (18) that extends at least substantially beyond the base body (10) in a radial direction (26) and is configured to ensure a distance extending perpendicularly to a processing axis between at least a large portion of the base body (10) and the material that is to be processed, **characterised in that** the processing unit (14) comprises at least two segment-wise arranged cutter elements (20) having a length l₁ which, in a state when connected with the base body (10), extends parallel to the axial direction (22) and along which the cutter elements (20) are partly embedded in the base body (10).

2. Surgical tool according to claim 1, **characterised in that** the processing unit (14) comprises maximally 50 cutter elements (20).

3. Surgical tool according to claim 1 or 2, **characterised in that** the at least two cutter elements (20) have a distance of at least 10 degrees from each other.

4. Surgical tool at least according to claim 1, **characterised in that** the at least two cutter elements (20) are made of a harder material than the base body (10).

5. Surgical tool at least according to claim 1, **characterised in that** the at least two cutter elements (20) are implemented at least substantially of at least one hard metal.

6. Surgical tool at least according to claim 1, **characterised in that** the at least two cutter elements (20) are implemented at least substantially of at least one ceramic material.

7. Surgical tool at least according to claim 1, **characterised in that** the at least two cutter elements (20) are implemented at least substantially ellipsoid-shaped.

8. Surgical tool at least according to claim 1, **characterised in that** the at least two cutter elements (20) are implemented by cutting teeth, which are at least partially arranged in a set manner.

9. Surgical tool according to at least one of the preceding claims, **characterised in that** the cutter elements (20) are connected with the base body (10) at least substantially by substance-to-substance bond.

10. Surgical tool according to at least one of the preceding claims, **characterised in that** the cutter elements (20) are at least substantially welded with the base body (10).

11. Surgical tool according to at least one of the preceding claims, **characterised in that** the cutter elements (20) are at least partially encompassed by the base body (10).

12. Surgical tool according to at least one of the preceding claims, **characterised in that** the base body (10) is at least substantially formed by a tube.

13. Surgical tool according to at least one of the preceding claims, **characterised in that** the cutter elements (20) are connected with the base body (10) such that they are at least substantially distributed evenly over a circumference of the base body (10).

## Revendications

1. Instrument chirurgical avec un corps de base (10) formé au moins sensiblement d'un corps en forme de cylindre creux, avec un élément de raccordement (12) et avec une unité de traitement (14),
l'unité de traitement (14) comprenant au moins une zone de traitement (16) qui s'étend dans une direction axiale (22) et au moins une zone de découpe (18) qui s'étend au moins sensiblement dans une direction radiale (26) au-delà du corps de base (10) et qui est configurée à au moins sensiblement assurer une distance s'étendant perpendiculairement à un axe de traitement entre au moins une grande partie du corps de base (10) et le matériau qui est à travailler,
**caractérisé en ce que** l'unité de traitement (14) comporte au moins deux éléments coupeurs (20) disposés par segments et ayant une longueur l₁ qui s'étend, dans un état d'être liés au corps de base (10), en parallèle à la direction axiale (22) et le long de laquelle les éléments coupeurs (20) sont partiellement enchâssés dans le corps de base (10).

2. Instrument chirurgical selon la revendication 1,
**caractérisé en ce que** l'unité de traitement (14) comprend maximalement 50 éléments coupeurs (20).

3. Instrument chirurgical selon la revendication 1 ou 2,
**caractérisé en ce que** les au moins deux éléments coupeurs ont une distance l'un de l'autre d'au moins 10 degrés.

4. Instrument chirurgical au moins selon la revendication 1,
**caractérisé en ce que** les au moins deux éléments coupeurs (20) sont formés d'un matériau plus dur que le corps de base (10).

5. Instrument chirurgical au moins selon la revendication 1,
**caractérisé en ce que** les au moins deux éléments coupeurs (20) sont formés au moins sensiblement d'au moins un métal dur.

6. Instrument chirurgical au moins selon la revendication 1,
**caractérisé en ce que** les au moins deux éléments coupeurs (20) sont formés au moins sensiblement d'au moins un matériau céramique.

7. Instrument chirurgical au moins selon la revendication 1,
**caractérisé en ce que** les au moins deux éléments coupeurs (20) sont implémentés au moins sensiblement en forme d'ellipsoïde.

8. Instrument chirurgical au moins selon la revendication 1,
**caractérisé en ce que** les au moins deux éléments coupeurs (20) sont implémentés de deux dents coupeuses, qui sont agencées au moins partiellement en avoyage.

9. Instrument chirurgical selon au moins l'une des revendications précédentes,
**caractérisé en ce que** les éléments coupeurs (20) sont liés avec le corps de base (10) au moins sensiblement par adhérence.

10. Instrument chirurgical selon au moins l'une des revendications précédentes,
**caractérisé en ce que** les éléments coupeurs (20) sont au moins sensiblement soudés avec le corps de base (10).

11. Instrument chirurgical selon au moins l'une des revendications précédentes,
**caractérisé en ce que** les éléments coupeurs (20) sont au moins partiellement entourés par le corps de base (10).

12. Instrument chirurgical selon au moins l'une des revendications précédentes,
**caractérisé en ce que** le corps de base (10) est au moins sensiblement implémenté d'un tuyau.

13. Instrument chirurgical selon au moins l'une des revendications précédentes,
**caractérisé en ce que** les éléments coupeurs (20) sont liés avec le corps de base (10) d'une telle manière qu'ils sont distribués sur une circonférence du corps de base (10) d'une façon au moins sensiblement bien régulière.
